# EUROPEAN PATENT SPECIFICATION

(11) **EP 2 590 582 B1**
(45) Date of publication and mention of the grant of the patent: **11.11.2015**
(21) Application number: 11732538.1
(22) Date of filing: 30.06.2011
(51) Int. Cl.: A61B 17/70

(54) **SPINAL STABILIZATION SYSTEM UTILIZING SCREW AND EXTERNAL FACET AND/OR LAMINA FIXATION**
WIRBELSÄULENSTABILISIERUNGSSYSTEM MIT EINER SCHRAUBE SOWIE EXTERNEN FACETTEN- UND/ODER LAMINAFIXERUNG
SYSTÈME DE STABILISATION SPINALE À VIS ET FIXATION SUR FACETTE EXTERNE ET/OU LAME VERTÉBRALE

(30) Priority: 08.07.2010 US 362334 P; 30.06.2011 US 201113173660
(43) Date of publication of application: 15.05.2013
(60) Divisional application: 15188588.6
(73) Proprietor: X-spine Systems, Inc., Miamisburg, OH 45342 (US)
(72) Inventor: KIRSCHMAN, David, Louis, Dayton OH 45429 (US)
(74) Representative: Rüfenacht, Philipp Michael
(86) International application number: PCT/US2011/042636
(87) International publication number: WO 2012/006216

(56) References cited:
- EP-A1- 2 111 811
- US-A1- 2005 131 406
- US-A1- 2006 200 149
- US-A1- 2006 293 663
- US-A1- 2007 043 357
- US-A1- 2009 112 264

## Description

### CROSS-REFERENCE TO RELATED APPLICATION

The present application claims priority to provisional U.S. Application Serial No. 61/362.334 filed July 8, 2010, and to U.S. Patent Application Serial No. 13/173,660, filed June 30, 2011, to which Applicant claims the benefit of the earlier filing dates.

### BACKGROUND OF THE INVENTION

### 1. Fied of the Invention

This invention relates to a spinal implant and, more particularly, to a spinal implant having a screw and a fixation component and variations thereof.

### 2. Description of the Related Art

The field of spinal implantation burgeons with devices and methods for the achievement of fixation between adjacent vertebrae. The most common devices currently used for fixation are pedicle screw systems. In a typical pedicle screw system, screws are placed into pedicles of adjacent vertebrae and are stabilized together using various separate rod or plate means. An emerging means for achieving fixation of adjacent vertebra is the use of trans-facet fixation. Several devices achieve fixation by placement of a screw or other means through the facet joint. This procedure has the advantage of being significantly less invasive than pedicle screw procedures because it does not require a separate rod or plate means and only requires two bilateral screws to achieve fixation per level, rather than four in a typical pedicle screw system. For these reasons, trans-facet fixation has been growing in popularity.

Some of the systems for bone fixation relating to facet fusion are shown or known from U.S. Patent Publications Nos. 2010/0114175 to McKay; 2010/0100135 to Phan; 2010/0094356 to Varela et al.; 2010/0087859 to Jackson, Jr.; 2010/0082065 to Butler et al.; 2010/0076490 to Greenwald et al.; 2009/0318980 to Falahee; 2009/0312763 to McCormack et al.; 2009/0312798 to Varela; 2009/0312800 to Chin et al.; 2009/0306671 to McCormack et al.; 2009/0299412 to Marino; 2009/0275954 to Phan et al.; 2009/0275992 to Phan et al.; 2009/0275993 to Phan et al.; 2009/0275994 to Phan et al.; 2009/0270929 to Suddaby; 2009/0264928 to Blain; 2009/0248082 to Crook et al.; 2009/0248089 to Jacofsky et al.; 2009/0234394 to Crook; 2009/0234397 to Petersen; 2009/0216273 to Cox; 2009/0192551 to Cianfrani et al.; 2009/0187219 to Pachtman et al.; 2009/0177205 to McCormack et al.; 2009/0163920 to Hochschuler et al.; 2009/0131986 to Lee et al.; 2009/0125066 to Kraus et al.; 2009/0112264 to Lins; 2009/0105819 to Barry; 2009/0099602 to Aflatoon; 2009/0093851 to Osman; 2009/0076551 to Petersen; 2009/0054903 to Falahee et al.; 2009/0036926 to Hestad; 2009/0036927 to Vestgaarden; 2009/0036986 to Lancial et al.; 2008/0275454 to Geibel; 2008/0262555 to Assell et al.; 2008/0255618 to Fisher et al.; 20008/0255619 to Schneiderman et al.; 2008/0255622 to Mickiewicz et al.; 2008/0255666 to Fisher et al.; 2008/0255667 to Horton; 2008/0234758 to Fisher et al.; 2007/0233092 to Falahee; 2007/0233093 to Falahee; 2007/0112428 to Lancial; 2006/0264953 to Falahee; 2006/0212034 to Triplett et al.; 2006/0200149 to Hoy et al.; 2006/0111779 to Petersen; 2006/0111780 to Petersen; 2005/0267480 to Suddaby; 2005/0149030 to Serhan et al.; 2005/0124993 to Chappuis; 2004/0254575 to Obenchain et al.; 2004/0087948 to Suddaby; and 2003/0208202 to Falahee, all of which are incorporated herein by reference and made a part hereof. Other systems are shown in U.S. Patent Nos. 6,648,893 issued to Dudasik; 7,608,094 issued to Falahee; 7,708,761 issued to Petersen; 7,563,275 issued to Falahee et al.; 7,699,878 issued to Pavlov et al.; 7,452,369 issued to Barry; 7,223,269 issued to Chappuis; 6,540,747 issued to Marino, 6,485,518 issued to Cornwall et al..

US 2009/0112264 to Lins discloses pedicle-based fixation systems and methods comprising a pedicle-based construct, such as a pedicle screw or pedicle anchor which is selectively anchored to transverse processes or the like. The pedicle-based construct is selectively coupled to a rod or other elongated member through a ball or the like disposed on one end of the pedicle-based construct. The rod or elongated member is coupled to a clamping mechanism which is configured to selectively clamp an inferior facet. The rod is thereby movably affixed to the pedicle-based construct. In one embodiment, the pedicle screw includes a tulip which receives a ball of the rod.

EP 2 111 811 to Biedermann Motech discloses an instrument for assembling a bone anchoring device, which includes a shank, a head being separate from the shank, and a receiving part having a first end, a second end and a longitudinal bore extending from the first to the second end, the head being positionable within the longitudinal bore at the second end. The bone anchoring device comprises - amongst others - a pressure element with a spherical surface which corresponds with a spherical outer surface of the head. This allows the head to be pivotable with respect to the pressure element in any direction prior to fixation.

US 2006/200149 to Hoy describes systems for attaching implants to bone, specifically to a polyaxial fastening apparatus. The apparatus comprises an implant, an orthopaedic fastener and a fixation member, wherein the fixation member may be a pedicle screw. The implant comprises a fixation portion, which is shaped to be attached to a semispherical resection of a pedicle, and a stem which connects the fixation portion to an articulation portion. The articulation portion comprises an articulation surface which is designed to articulate with a superior facet of an immediately inferior vertebra. The fastener comprises an interpositional member and a compression member, wherein the compression member is designed to be advanced over a proximal end of the fixation member to press the interpositional member into an interpositional surface of the implant. When the interpositional member is relatively compressed, the fastener is in a locked configuration.

US 2005/131406 to Reiley et al. describes systems and prostheses to help establish a desired anatomy to a spine. In one embodiment, an artificial caudal facet joint prosthesis is provided which is configured to replace a natural facet joint. The prosthesis comprises an artificial facet joint element which articulates with the inferior half of the facet joint, which may comprise the natural inferior half of the facet joint on the vertebral body below. The prosthesis further comprises a fixation element which is connected by means of a polyaxial connection to the facet joint element. The polyaxial connection permits the rotation of facet joint element with respect to the fixation element.

US 2006/023663 to Walkenhorst et al. describes a dynamic stabilization system for the spine comprising a bone engaging fastener and a deflectable or bumper element fastened to the bone engaging fastener. The deflectable element is configured and arranged to contact a portion of an adjacent vertebra, such as the facet. The bone engaging fastener includes a shank having bone engaging threads, a threaded stem and a nut configured to engage the threaded stem. The deflectable element comprises an opening to receive the threaded stem therethrough and the nut bears on the deflectable element to mount it on the fastener. Further, the bone engaging fastener may comprise a shoulder onto which the deflectable element is pressed by means of the nut such as to be locked between the shoulder and the nut.

US 2007/043357 to Kirschmann describes a spinal fixation assembly and capless multi-axial screw system comprising a receiver having a rotary lock having a plurality of channels which urge and lock an elongated member to a screw using a bayonet type connection.

US 2006/241597 to Mitchel et al. discloses interspinous process implants comprising a facet joint spacer which is hingedly connected to a lateral mass plate. An interior surface of the facet joint spacer includes a plurality of protrusions which penetrate a superior facet of the targeted facet joint. The mass plate may be positioned outside of the facet joint and comprises a bore for a bone screw. The bone screw is held in an angled position within the bore by means of a locking plate with a keel which Is arranged on top of the screw head.

US 2008/0255622 to Mickiewicz et al. discloses a spinal implant for stabilization and/or fusion of facets. The Implant includes an elongate member coupled to a stabilization member so as to allow a polyaxial motion of the stabilization member relative to the elongate member. The elongate member thereby passes through an opening provided in the stabilization member. The stabilization member comprises a plurality of tines adapted to pierce an outer portion of the facet joint. The stabilization member may be configured to include at least one lateral extension in a plate-like configuration. The lateral extension includes at least one opening which is configured to receive a fixation member.

US 5,429,639 to Tornier SA relates to spine fixators for aligning and holding a backbone. The fixators comprise a ring which is square in its upper part and rounded in its lower part and having an opening along its horizontal axis to receive U-shaped profiles. The ring further comprises a tapped opening in the vertical axis to receive a screw. A further horizontal opening is provided perpendicular to the first opening to receive a connection rod. A screw having a self-tapping thread and a spherical head is used to attach the fixator on vertebrae. A threaded cap cooperates with the tapped opening to block the screw head against the ring.

FR 2 827 499 to Graf Henry describes a vertebral fixation assembly. The fixation assembly comprises at least one fix element which may be connected to a vertebra, one mobile element which may be movable in relation to the fix element as well as an intermediary element which enables a movement between the fix element and the mobile element. Once assembled, the mobile element has three degrees of freedom relative to said mobile element but is translationally fix thereto. The fix element is preferably in the form of a pedicle screw having a spherical head and a threaded shank. The spherical head comprises a flat circumferential portion with a diameter corresponding to the opening of a spherical receptacle of the intermediary element. Introduction of the head into the receptacle is possible by orienting the head in such a way that the flat portion is aligned with the opening. With a set screw re-alignment of the flat portion with the opening is prevented.

There are significant disadvantages, however, to trans-facet fixation. The procedure is technically demanding, particularly in the so-called translaminar approach where the fixation screw must be placed very close to critical nerve elements. Compared to pedicle anatomy, which is relatively constant from patient-to-patient, facet anatomy is quite variable and can lead to variations in screw purchase and fixation strength. Furthermore, the facet joints are rather small compared to the pedicles are far more prone to fracturing than pedicles during screw placement.

Therefore, what is needed is a new apparatus, system and method that preserves the minimally invasive nature of trans-facet fixation while mitigating the problems of anatomy variation, facet fracture, and technical difficulty.

### SUMMARY OF THE INVENTION

The facet joint has two general parts, the internal articular surface which functions to slide against the adjacent facet joint of an adjacent vertebra, and a bony external surface which arises from the lamina or posterior portion of the vertebrae. This external surface is comprised of cortical bone. This external portion is strong, easily accessible, and safely away from critical nerve elements. It is the intention of one embodiment to use this surface as a fixation point. It is further the intention of one embodiment to use the pedicle of the adjacent vertebra as the other fixation point.

One object of one embodiment is to provide an improved spinal stabilization system utilizing screw and external facet and/or laminar fixation.

Another object of another embodiment is to provide a fixation component that can be removably mounted on a screw.

Still another object of another embodiment is to provide a surgical implant having a fixation component that is fixed relative to the screw.

Still another object of another embodiment is to provide a fixation component that can move polyaxially relative to the screw.

Still another object is to provide various apparatus and means for securing the fixation component to the screw and for providing polyaxial movement of the fixation component relative to the screw.

In one aspect, one embodiment of the invention provides a surgical implant for support of spinal vertebrae, the surgical implant comprising a screw element having a threaded shank for screwing into a pedicle of a first vertebra, a fixation component that is moveably secured to the screw element, the fixation component having a first end for receiving the screw element and a fixation surface adapted to engage or attach to at least one of an external facet or laminar surface of a second vertebrae.

Preferred embodiments of this surgical implant comprise the following features either alone or in combination:
- Said screw element comprises a polyaxial portion for permitting said fixation component to move polyaxially on said screw element until said fixation component is fixed thereto, wherein the polyaxial portion comprises a polyaxial mount located on said screw element, said polyaxial mount being adapted to polyaxially receive said first end of said fixation component.
- Said polyaxial mount comprises a spherical head, wherein preferably said spherical head is defined by a compressive member removably received on said screw element.
- Said fixation component comprises a linear or curvilinear body having said fixation surface, wherein said fixation surface comprises a plurality of teeth for engaging said at least one of said external facet or said laminar surface of said second vertebra.
- Said screw element and said fixation component are adapted to permit at least one of polyaxial motion of said fixation component relative to said screw element or polyaxial motion of said screw element relative to said fixation component, wherein said at least one of polyaxial motion of said fixation component relative to said screw element or said polyaxial motion of said screw element relative to said fixation component is achieved by adapting said screw element and said fixation component to define a ball and socket coupling. Preferably, said ball is located on said screw element and said socket is located on said fixation component. Alternatively, said ball is located on said fixation component and said socket is located on said screw element.
- Said fixation component has a bone-engagement portion and a locking portion for locking onto said screw element, said bone-engagement portion having an engagement surface for engaging bone, said bone-engagement portion being generally circular or elongated and generally conforming to a local anatomy.
- Said spinal implant further comprises a lock for locking said fixation component to said screw element, wherein said lock comprises at least one of a set screw, cap, nut or bolt.

The invention provides a fixation component that is moveably secured to a screw element, the fixation component comprising, a first end secured to the screw element and a fixation surface adapted to engage or attach to at least one of an external facet or laminar surface of a second vertebrae.

Preferred embodiments of this fixation component comprise the following features either alone or in combination:
- Said fixation component further comprises an aperture for receiving a threaded portion of said screw element, said threaded portion being adapted to receive a nut for fixing said fixation component to said screw element after said screw element is screwed into said second vertebra.
- Said second vertebra is an immediately adjacent vertebra, wherein preferably, said first end of said fixation component has a fixation component wall that defines a generally spherical fixation component receiving opening for receiving a portion of said screw element and a generally spherical member associated therewith.
- Said generally spherical member is a compression element.
- Said generally spherical member comprises a split ball.
- Said fixation component comprises a threaded area associated with said first end for receiving an open nut, said open nut having an inner wall that defines a nut receiving area that cooperates with said fixation component receiving opening to define a receiving area for receiving a portion of the screw element to facilitate locking said fixation component thereon.
- Said portion of said screw element comprises a polyaxial portion for permitting said fixation component to move polyaxially on said screw element until said fixation component is fixed thereto.
- Said polyaxial portion comprises a polyaxial mount located on said screw element, said polyaxial mount being adapted to polyaxially receive said first end of said fixation component.
- Said fixation component comprises a linear or curvilinear body having said fixation surface.
- Said fixation surface comprises a plurality of teeth for engaging said at least one of said external facet or said larriinar surface of said second vertebra.
- Said fixation surface comprises a curved, concave or multi-angular surface to match the anatomy of said at least one of said external facet or said laminar surface of said second vertebra.
- Said fixation component is adapted to permit at least one of polyaxial motion of said fixation component relative to said screw element or polyaxial motion of said screw element relative to said fixation component.
- Said fixation component comprises a socket or ball and said screw element comprises a ball or socket, respectively, said ball being received in said socket to permit said at least one of polyaxial motion of said fixation component relative to said screw element or said polyaxial motion of said screw element relative to said fixation component.
- Said ball is located on with said screw element and said fixation component comprises said socket.
- Said ball is located on or integral with said fixation component and said screw element comprises said socket.
- Said screw element comprises a head having a socket and said fixation component comprises a ball or spherical component for mounting in said socket, said spinal implant further comprising a lock for locking said ball in said socket. Preferably, said lock is at least one of a set screw, a cap or an interference or frictional fit between said ball and said socket.
- Said fixation component has a bone-engagement portion and a locking portion for locking onto said screw element, said bone-engagement portion having an engagement surface for engaging bone, said bone-engagement portion being generally circular or elongated and generally conforming to a local anatomy.

These and other objects and advantages will be apparent from the following description, the accompanying drawings and the appended claims.

### BRIEF DESCRIPTION OF THE DRAWINGS

The invention is illustrated in the figures 8A-8B and 9. The remaining figures illustrate examples that are useful for understanding the invention.
Fig. 1 is a view of one example showing a fixation component attached to a screw element;
Fig. 2 is an exploded view of the embodiment shown in Fig. 1;
Fig. 3 is a view showing various details of the screw element;
Fig. 4 is a view of the fixation component shown in Fig. 1 showing an angled profile and plurality of engagement teeth;
Fig. 5 is a plan view of the fixation component shown in Fig. 1;
Fig. 6 is a view of another example of the fixation component showing an elongate portion having a curved fixation surface;
Fig. 7 is a view showing further details of the fixation component shown in Fig. 6;
Fig. 8A is a view of embodiment illustrating a fixation component that cooperates with a split ball and locking nut having a inner spherical surface to permit polyaxial movement of the fixation component;
Fig. 8B is a sectional view taken along the line 8B-8B in Fig. 8A;
Fig. 9 is an exploded view of the embodiment shown in Figs. 8A and 8B;
Figs. 10A-10F are various examples illustrating polyaxial movement of the fixation component relative to a polyaxial screw used with or without a retainer or housing and with various means for fixing or locking the fixation component to the polyaxial screw;
Fig. 11 is a view illustrating a placement of the spinal implant to fix a facet joint; and
Figs. 12A-12B are further enlarged fragmentary views of a portion of the view in Fig. 11 further illustrating the use of the fixation component.

### DETAILED DESCRIPTION OF PREFERRED EMBODIMENTS

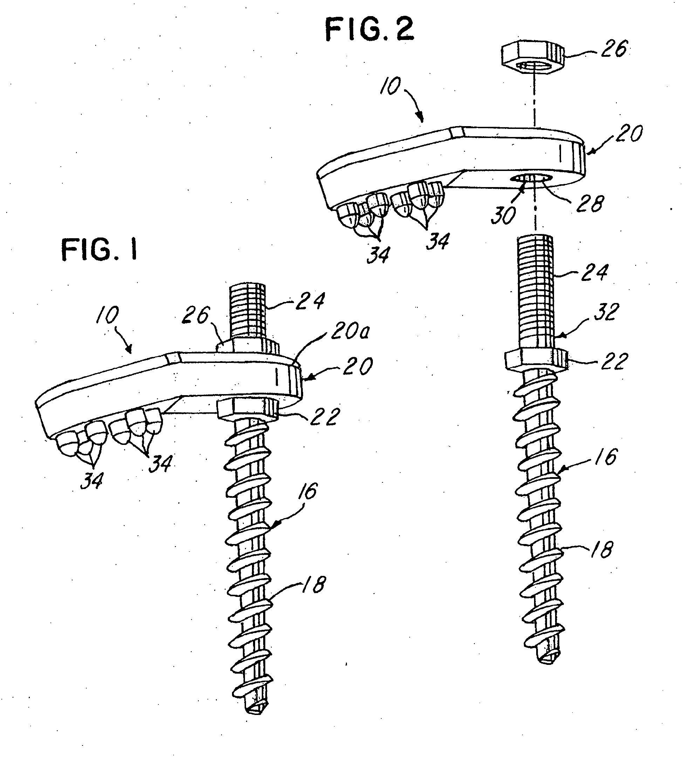

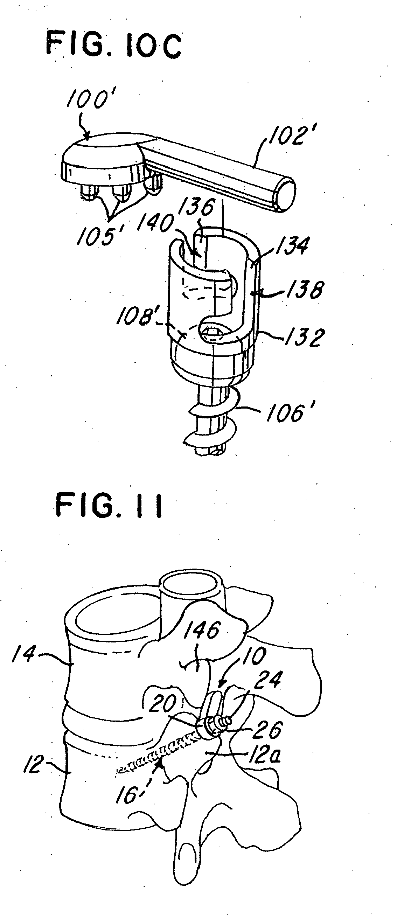

Referring now to Figs. 1-7, a surgical implant 10 is shown. In the illustration being described, the surgical implant 10 is for the support of spinal vertebrae, such as a first vertebra 12 (Fig. 11) and a second vertebra 14. The surgical implant 10 (Figs. 1-5) is adapted to be secured to, for example, the first vertebra 12 and adapted to engage or attach to at least one of an external facet or laminar surface 14b of the second vertebra 14.

In the illustration being described, the surgical implant 10 comprises a screw element 16 (Figs. 1-2) having a threaded shank 18 for screwing into a facet 12a (Fig. 11) of the first vertebra 12. The surgical implant 10 further comprises a fixation component 20 that is moveably secured to the screw element 16. The fixation component 20 comprises a first end 20a and a second end 20b.

The screw element 16 comprises a nut 22 that is integrally formed therewith and a threaded portion 24 that threadably receives a nut 26 best shown in Figs. 1 and 2. Note that the fixation component 20 comprises an inner wall 28 that defines an aperture or bore 30 for receiving the threaded portion 24 as best illustrated in Fig. 2. In this regard, it should be understood that the inner wall 28 comprises a circumference that is slightly larger than the threaded portion 24 so that the threaded portion 24 can be inserted through the aperture 30.

The screw element 16 comprises a connection portion 32 which in the embodiment of Figs. 1 and 2 is defined by the threaded portion 24. In the illustration being described in Figs. 1 and 2, the threaded portion 24 is at an opposite end of the threaded shank 18 and is adapted and dimensioned to removably receive the fixation component 20. The threaded portion 24 is coaxial with the threaded shank 18.

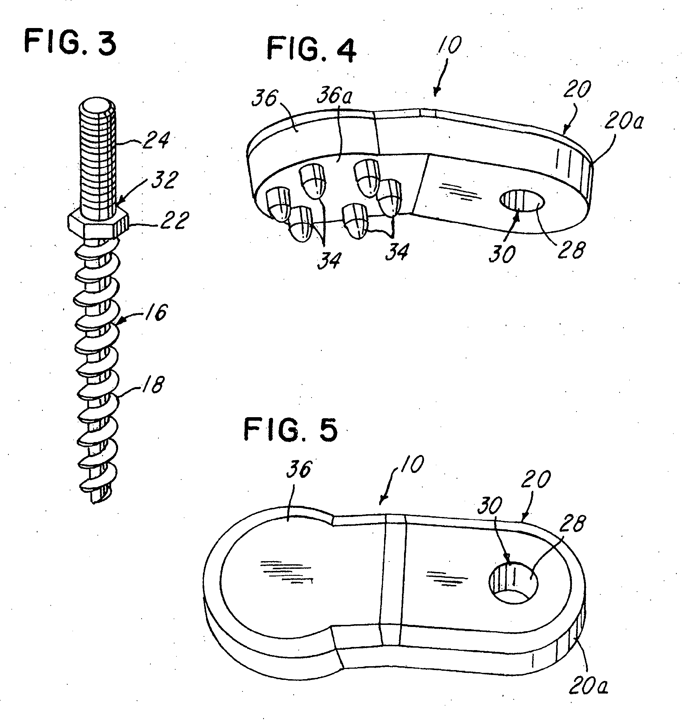

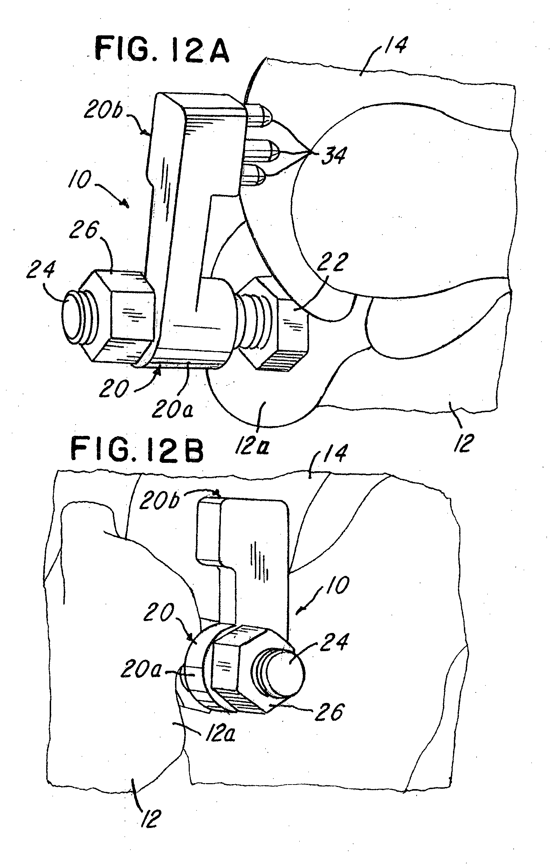

The second end 20b of the fixation component 20 has a fixation or frictional surface 36a of a body 36 that defines the fixation component 20. The plurality of engagement teeth 34 are adapted to engage or attach to at least one of the external facet or laminar surface 14b of the second vertebra 14. In regards to the engagement teeth 34 (Figs. 1 and 4), it should be understood that fewer or more teeth may be used, or alternatively, a serrated or other type of machined surface on the frictional surface 36a of the body 36 may be provided to facilitate frictional engagement of the bone. In the illustration being described, it should be understood that the second vertebra 14 is immediately adjacent the first vertebra 12 as shown in Figs. 11 and 12A-12B; however, it should be understood that the fixation component 20 could be adapted to traverse a greater distance such that it can engage a vertebra (not shown) that is not immediately adjacent to the first vertebra 12.

Returning to the embodiment of Figs. 1-5, it should be understood that while the fixation component 20 is shown as having a body having a generally angled profile, the body could comprise a linear shape, a curvo-linear shape, a concave shape, semi-spherical shape, multi-angular shape or other shape or surface to generally correspond to or match an anatomy of the at least one of the external facet or laminar surface 14b of the second vertebra 14.

After the surgeon screws the screw element 16 into the facet 12a of the first vertebra 12, as shown in Figs. 11 and 12A-12B, the fixation component 20 is mounted or guided onto the screw element 16 by inserting the threaded portion 24 through the aperture 30. The nut 26 is then threadably mounted on the threaded portion 24 to secure the fixation component 20 to the connection portion 32 of the screw element 16.

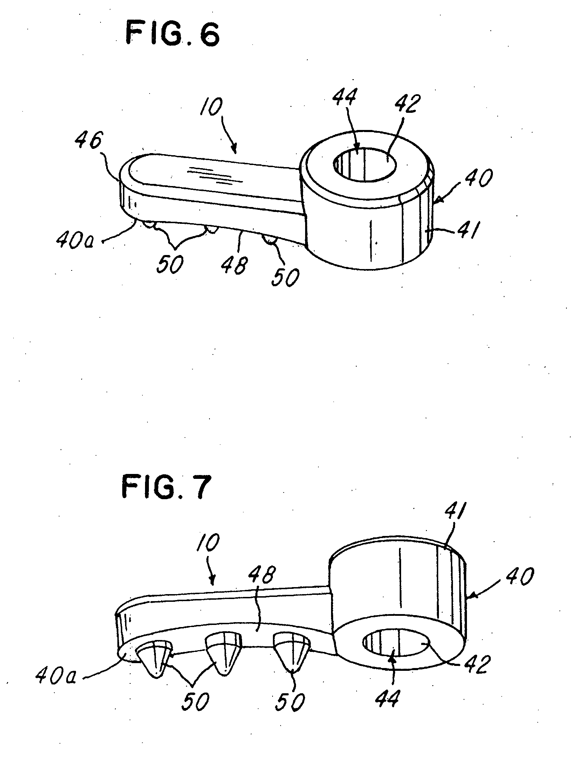

Figs. 6 and 7 illustrate another fixation component 40 that could be mounted on the screw element 16. In this embodiment, the fixation component 40 has a first portion 41 having an internal wall 42 that defines an aperture 44 for receiving the connection portion 32, which in the illustration in Figs. 1-5 is the threaded portion 24. Note that the fixation component 40 in the embodiment of Figs. 6 and 7 has an elongated portion 46 having a curved surface 48 that is adapted or shaped to match a desired anatomy. In this illustration, the elongated portion 46 extends generally radially from an axis defined by the aperture 44 and comprises a fixation surface 40a having a plurality of engagement teeth 50 that are aligned linearly. As with the embodiment of Figs. 1-5, the embodiment of the fixation component 40 in Figs. 6 and 7 would be mounted on the screw element 16 and secured thereto with the nut 26.

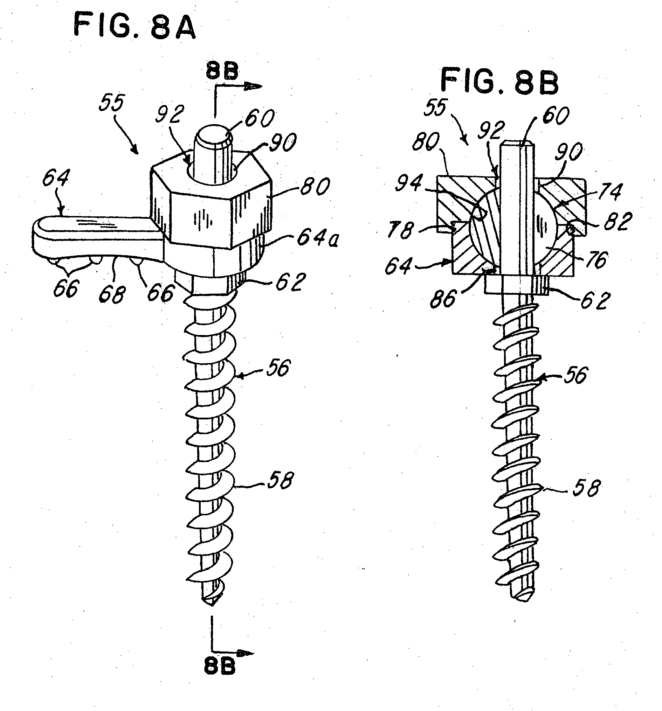

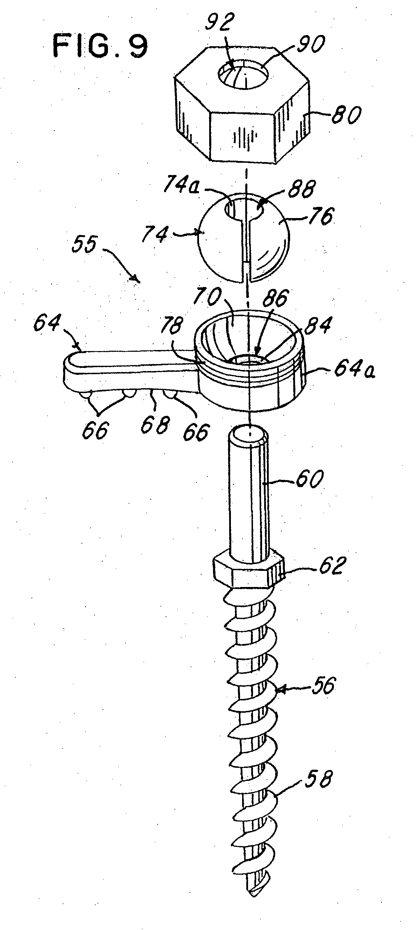

It is desired to provide a fixation component that moves polyaxially relative to the screw element 16. Figs. 8A-10F illustrate various embodiments for permitting such polyaxial movement. Figs. 8A, 8B and 9 illustrate an embodiment of a surgical implant 55 that comprises a screw element 56 having a threaded shank 58 and a connection potion 60 that is generally cylindrical and coaxial with the threaded shank 58 as shown. As with the previous embodiments, the screw element 56 comprises an integral nut 62 for screwing the screw element 56 into bone.

In the illustration being described, the surgical implant 55 comprises a fixation component 64 having a plurality of teeth 66 that are integral with a curved surface 68 similar to the curved surface 48 in the embodiment shown in Figs. 6 and 7. Note that a first end 64a of the fixation component 64 comprises an interior spherical surface that is adapted and dimensioned to receive a compression element 74 in the form of a split ball 76. The first end 64a also comprises external or male threads 78 that are adapted to receive a nut 80 having female threads 82 (Fig. 8B) that threadably receive the male threads 78.

In the illustration being shown in Figs. 8A, 8B and 9, the fixation component 64 comprises an interior wall 84 defining a bore 86 for receiving the connection portion 60. After the screw element 56 is situated into bone, such as the first vertebra 12 in Fig. 11, the fixation component 64 (Fig. 9) receives the connection portion 60 which receives the split ball 76. In this regard, the split ball 76 has an interior wall 74a that defines a bore 88 that is adapted to receive the connection portion 60.

After the fixation component 64 is properly positioned in the patient and the split ball 76 is positioned onto the connection portion 60, the nut 80 receives the connection portion 60 as shown. Note that the nut 80 comprises an internal wall 90 that defines an internal bore 92 for receiving the connection portion 60. Note also that the nut 80 has an interior spherical wall 94 (Fig. 8B) that is adapted and dimensioned to complement the shape of the split ball 76 as best illustrated in Fig. 8B.

In the illustration being described, after the screw element 56 is screwed into bone, the bore 86 of fixation component 64 is received on the connection portion 60 along with the split ball 76. The connection portion 60 is also received in the internal bore 92 of the nut 80, and as the nut 80 is screwed onto the fixation component 64, as best illustrated in Fig. 8B, the interior spherical wall 94 cooperates with the spherical wall 70 of the fixation component 64 to compress the split ball 76 which causes it to compress against the connection portion 60, thereby locking the fixation component 64 to the screw element 56.

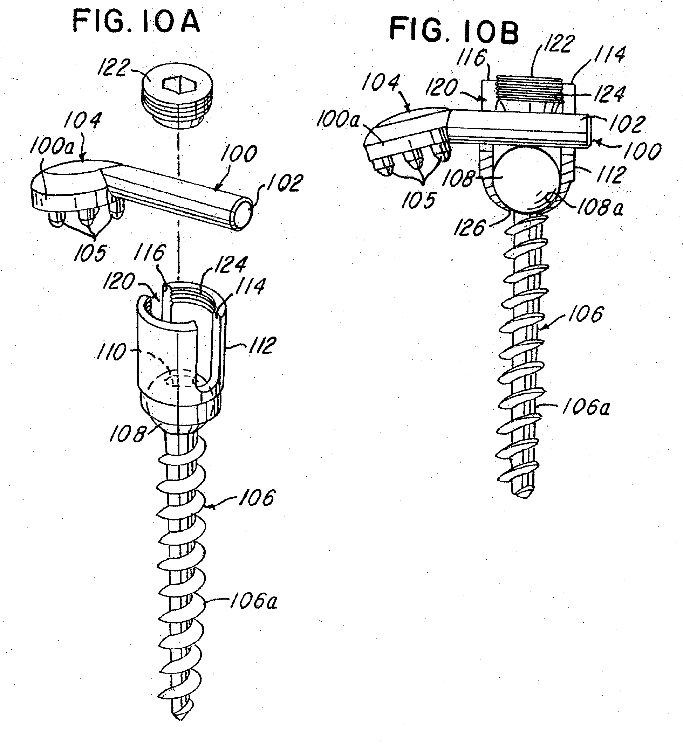

The examples described below are not claimed. They provide information that is useful for understanding the invention. For example, Fig. 10A illustrates another example wherein a fixation component 100 has an engagement portion 104 that engages the at least one of the external facets or laminar surfaces of the second vertebra 14. In this regard, note that the embodiment comprises a polyaxial screw 106 having a spherical or polyaxial head 108 and a hex-female slot 110 for receiving a tool (not shown) for rotatably driving the polyaxial screw 106.

As illustrated in Fig. 10A, the polyaxial screw 106 comprises the spherical or polyaxial head 108 that is adapted to be received in a receiver 112 having a pair of walls 114 and 116 that define a channel 120. In this regard, the receiver 112 may comprise features illustrated in U.S. Patents 7,717,943; 7,662,172; 7,604,655; 7,686,835 and U.S. Patent Publication Nos. 2010/0204738; 2006/0155278; 2007/0123862; 2007/0093827; 2007/0123867; 2008/0071277; 2008/0097457; 2008/0249576; 2010/0063553; 2010/0179603 and 2010/0152788.

Note in the illustration shown in Fig. 10A that the channel 120 receives an elongated rod or cylindrical portion 102 of the fixation component 100. The embodiment illustrated in Figs. 10A-10B comprises a threaded cap 122 that is threadably received in the female threads 124 in the receiver 112. The receiver 112 comprises a wall 126 (Fig. 10B) that defines a bore 128 for receiving the threaded shank 106a of the polyaxial screw 106.

After the surgeon screws the polyaxial screw 106 into bone, such as the first vertebra 12 (Fig. 11), the receiver 112 (Fig. 10A) is polyaxially positioned and the cylindrical portion 102 of the fixation component 100 is situated in the channel 120 and positioned such that the plurality of teeth 105 on end 100a engage at least one of the facet or laminar surface 14a of the second vertebra 14. Thereafter, the threaded cap 122 is threadably received in the female threads 124 of the receiver 112 and screwed thereon until the cylindrical portion 102 engages the polyaxial head 108 of the polyaxial screw 106, thereby locking the fixation component 100 into the receiver 112 and on the polyaxial screw 106 and fixing the facet joint associated with the first and second vertebrae 12 and 14. Although not shown, an external, rather than internal, cap could be used in this embodiment.

Fig. 10C illustrates still another example of a polyaxial implant 130. In this embodiment, those parts that are the same or similar to the parts of the embodiments in Fig. 10A-10B are identified with the same part number, except that a prime mark ("'") has been added to the part numbers of the embodiment in Fig. 10C. Note in this embodiment that the locking is accomplished utilizing a receiver 132 having a bayonet-type connection. In this regard, note that the receiver 132 comprises walls 134 and 136 that define two generally L-shaped channels 138 and 140, respectively. In this embodiment, the receiver 132 receives the polyaxial screw 106'and the surgeon screws the polyaxial screw 106' into the first vertebra 12. The surgeon polyaxially moves the receiver 132 relative to the polyaxial head 108' until the fixation component 100' is situated in the proper position such that the plurality of teeth 105'are in engagement with the facet. Thereafter, the surgeon rotates the receiver 132 relative to the polyaxial screw 106' and fixation component 100' which causes the cylindrical portion 102' to be driven downward (as viewed in Fig. 10C) and against the head 108' of the polyaxial screw 106'. In this regard, the operation of the capless embodiments shown in Fig. 10C is substantially the same or similar to and may include the same features as the devices shown in U.S. Patents 7,717,943; 7,662,172; 7,604,665; 7,686,835 and U.S. Patent Publication Nos. 2010/0204738; 2006/0155278; 2007/0123862; 2007/0093827; 2007/0123867; 2008/0071277; 2008/0097457; 2008/0249576; 2010/0063553; 2010/0179603 and 2010/0162788.

It should be understood that the examples shown in Fig. 10A-10C illustrate receivers 112 and 132 wherein the polyaxial screw 106 and 106' is loaded from the top (as viewed in Figs. 10A-10C) such that the polyaxial head 108 is seated against the spherical wall 108a (Fig. 10B). It should be understood, however, that the screw may be "bottom loaded" and similar to one or more of the devices shown in one or more of the above-referenced patents or publications earlier herein.

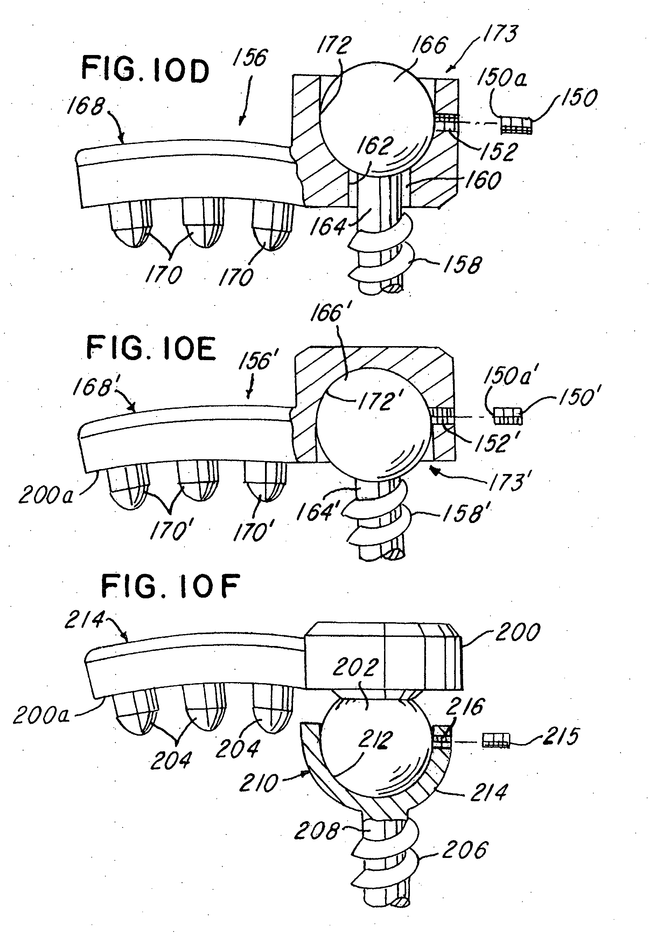

Fig. 10D illustrates yet another example showing a fixation component 168 similar to the embodiment of Figs. 8A, 8B and 9 except that rather than a nut 80, a set screw 150 is used to lock the fixation component 168 to a polyaxial screw 158. This embodiment is similar to that shown in Fig. 9, except that the fixation component 168 does not utilize the nut 80 in cooperation with the male thread 78. In this regard, the implant 156 in Fig. 10D comprises a polyaxial screw 158 that is received in a bore 160 defined by an internal wall 162 that receives the shank 164 of the polyaxial screw 158. Note that the polyaxial screw 158 comprises the generally cylindrical head 166. The implant 156 comprises a fixation component 168 having the plurality of teeth 170 and a spherical shaped wall 172 that defines a socket 173 that receives the generally cylindrical head 166 as shown. After the surgeon screws the polyaxial screw 158 into the first vertebra 12, the fixation component 168 is polyaxially moved or situated against at least one of the external facet or laminar surface of the second vertebra 14. After the fixation component 168 is positioned where desired, the set screw 150 is screwed into the threaded aperture 152 until an end 150a engages the cylindrical head 166 of the polyaxial screw 158, thereby locking the fixation component 168 to the polyaxial screw 158.

The example shown in Fig. 10E, with the like parts being identified with like part numbers except a prime mark ("'") is added to those in Fig. 10E, a "bottom-loaded" fixation component 168', rather than the top-loaded component 168 shown in Fig. 10D.

Fig. 10F illustrates still another example of a ball and socket arrangement wherein the ball is located on or integral with the fixation component and the socket is situated in the screw head of a polyaxial screw. Like the example illustrated in Fig. 10D, this embodiment permits polyaxial movement of the fixation component relative to the screw head.

In this example, a fixation component 200 comprises an integral ball 202 and, like the prior embodiments, has a fixation surface 200a having a plurality of engagement teeth 204 as shown. The spinal implant in the embodiment of Fig. 10F further comprises a polyaxial screw 206 having a threaded shank 208 and a head portion 210. Note that the head portion 210 comprises an inner spherical surface 212 that defines a receiving area or socket 214 for receiving the ball 202. A set screw 215 is threadably screwed into the wall in the head 216 in order to lock the fixation component 200 to the screw 206.

In the illustrations shown in Figs. 10D, 10E and 10F, a ball and socket arrangement is used with a set screw, such as set screw 150, to lock the fixation component relative to the screw element. However, it should be understood that other means or apparatus could be used for locking these components together, such as providing an interference or frictional fit between the two elements; provided, however, that the components do not move relative to each other after placement in the patient.

Advantageously, the embodiments being described provide a surgical implant having an anatomically-shaped fixation element which on one end is secured to the screw element and on the other rigidly attaches to the external, non-articular, surface of the facet joint of the adjacent vertebra, such as the second vertebra 14. The embodiments described enable the implants to comprise means of attachment between the screw element and the fixation element and which provide the ability to vary the angles between the screw element and the fixation components to conform to regional anatomy of the patient.

## Claims

1. A surgical implant for support of spinal vertebrae (12, 14) said surgical implant (55) comprising:
a) a screw element (56) having a threaded shank (58) for screwing into a pedicle of a first vertebra (12) and a connection portion (60) comprising an attachment shaft that is integral and contiguous with said threaded shank (58);.
b) a fixation component (64) that is moveably secured to said screw element (56), said fixation component (64) having a first end (64a) for receiving said screw element (56) and a fixation surface (68) having a shape or surface adapted to engage or attach to at least one of an external facet or laminar surface (14b) of a second vertebra (14), said first end (64a) of said fixation component (64) having a fixation component wall (84) that defines a fixation oomponent receiving opening;
c) a compression element (74) comprising a bore (88) for receiving said oonnection portion (60);
**characterized In that** said fixation component (64) comprises a threaded area (78) associated with said first
end (64a) defining a nut receiving area for receiving a nut (80), said nut receiving area cooperating with said fixation component receiving opening to define a compression element receiving area for receiving said compression element (74), said nut (80) engaging said compression element (74) to compress against said attachment shaft (60) of said screw element (56) when said nut (80) is screwed onto said threaded area (78) of said first end (64a), thereby fixing said fixation component (64) to said screw element (56).

2. The surgical implant as recited in any of the preceding claims wherein said second vertebra (14) is an immediately adjacent vertebra.

3. The surgical implant as recited In any of claims 1 or 2, wherein said fixation surface (68) comprises a plurality of engagement teeth (66) for engaging said at least one external facet or laminar surface (14b) of said second vertebra (14).

4. The surgical implant as recited in any of the preceding claims wherein said fixation component (64) comprises a linear or curvilinear body having said fixation surface (68).

5. The surgical implant as recited in any of the preceding claims wherein said fixation surface (68) comprises a curved, concave or multi-angular surface to match an anatomy of said at least one of said external facet or said laminar surface (14b) of said second vertebra (14).

## Patentansprüche

1. Chirurgisches Implantat zum Stützen von Rückenwirbeln (12, 14), wobei das chirurgische Implantat (55) Folgendes umfasst:
a) ein Schraubenelement (56) mit einem Gewindeschaft (58) zum Einschrauben in ein Pedikel eines ersten Wirbels (12) und einem Verbindungsabschnitt (60), der einen Anbringschaft umfasst, der mit dem Gewindeschaft (58) integral ist und an diesen angrenzt,
b) eine Fixierungskomponente (64), die beweglich an dem Schraubenelement (56) befestigt ist, wobei die Fixierungskomponente (64) ein erstes Ende (64a) zur Aufnahme des Schraubenelements (56) und eine Fixierungsfläche (68) mit einer Form oder Oberfläche hat, die geeignet ist, eine äußere Facette und/oder eine laminare Fläche (14b) eines zweiten Wirbels (14) in Eingriff zu nehmen oder daran angebracht zu werden, wobei das erste Ende (64a) der Fixierungskomponente (64) eine Fixierungskomponentenwand (84) hat, die eine Fixierungskomponentenaufnahmeöffnung definiert,
c) ein Kompressionselement (74), das eine Bohrung (88) zur Aufnahme des Verbindungsabschnitts (60) umfasst,
**dadurch gekennzeichnet, dass**
die Fixierungskomponente (64) einen dem ersten Ende (64a) zugeordneten Gewindebereich (78) umfasst, der einen Mutteraufnahmebereich zur Aufnahme einer Mutter (80) definiert, wobei der Mutteraufnahmebereich mit der Fixierungskomponentenaufnahmeöffnung zusammenwirkt, um einen Kompressionselementaufnahmebereich zur Aufnahme des Kompressionselements (74) zu definieren, wobei die Mutter (80) das Kompressionselement (74) in Eingriff nimmt, um es gegen den Anbritigschaft (60) des Schraubenelements (56) zu drücken, wenn die Mutter (80) auf den Gewindebereich (78) des ersten Endes (64a) geschraubt wird, wodurch die Fixierungskomponente (64) an dem Schraubenelement (56) fixiert wird.

2. Chirurgisches Implantat nach einem der vorhergehenden Ansprüche, wobei der zweite Wirbel (14) ein unmittelbar benachbarter Wirbel ist.

3. Chirurgisches Implantat nach Anspruch 1 oder 2, wobei die Fixierungsfläche (68) eine Vielzahl von Eingriffszähnen (66) zur Ineingriffnahme der äußeren Facette und/oder der laminaren Fläche (14b) des zweiten Wirbels (14) umfasst.

4. Chirurgisches Implantat nach einem der vorhergehenden Ansprüche, wobei die Fixierungskomponente (64) einen linearen oder kurvenförmigen Körper umfasst, der die Fixierungsfläche (68) hat.

5. Chirurgisches Implantat nach einem der vorhergehenden Ansprüche, wobei die Fixierungsfläche (68) eine gekrümmte, konkave oder mehrwinklige Fläche umfasst, um mit einer Anatomie der äußeren Facette und/oder der laminaren Fläche (14b) des zweiten Wirbels (14) zusammenzupassen.

## Revendications

1. Implant chirurgical servant au support de vertèbres spinales (12, 14), ledit implant chirurgical (55) comprenant :
a) un élément formant vis (56) comportant une tige filetée (58) destinée à être vissée dans un pédicule d'une première vertèbre (12) et une partie de raccordement (60) comprenant une tige de fixation qui fait partie intégrante de ladite tige filetée (58) et est adjacente à celle-ci ;
b) un composant d'assujettissement (64) qui est attaché de façon mobile audit élément formant vis (56), ledit composant d'assujettissement (64) comportant une première extrémité (64a) destinée à recevoir ledit élément formant vis (56) et une surface d'assujettissement (68) présentant une forme ou une surface conçue pour s'accoupler avec une facette externe et/ou une surface laminaire (14b) d'une seconde vertèbre (14) ou se fixer à celle-ci, ladite première extrémité (64a) dudit composant d'assujettissement (64) comportant une paroi de composant d'assujettissement (84) qui définit une ouverture de réception de composant d'assujettissement ;
c) un élément de compression (74) comprenant un orifice (88) destiné à recevoir ladite partie de raccordement (60) ;
**caractérisé en ce que**
ledit composant d'assujettissement (64) comprend une région filetée (78) associée à ladite première extrémité (64a) définissant une région de réception d'écrou destinée à recevoir un écrou (80), ladite région de réception d'écrou coopérant avec ladite ouverture de réception de composant d'assujettissement afin de définir une région de réception d'élément de compression destinée à recevoir ledit élément de compression (74), ledit écrou (80) s'accouplant avec ledit élément de compression (74) afin de le comprimer contre ladite tige de fixation (60) dudit élément formant vis (56) lorsque ledit écrou (80) est vissé sur ladite région filetée (78) de ladite première extrémité (64a), ledit composant d'assujettissement (64) étant ainsi assujetti audit élément formant vis (56).

2. Implant chirurgical selon l'une quelconque des revendications précédentes, dans lequel ladite seconde vertèbre (14) est une vertèbre directement adjacente.

3. Implant chirurgical selon l'une quelconque des revendications 1 et 2, dans lequel ladite surface d'assujettissement (68) comprend une pluralité de dents d'accouplement (66) destinées à former un accouplement avec ladite ou lesdites facette externe et/ou surface laminaire (14b) de ladite seconde vertèbre (14).

4. Implant chirurgical selon l'une quelconque des revendications précédentes, dans lequel ledit composant d'assujettissement (64) comprend un corps rectiligne ou curviligne comportant ladite surface d'assujettissement (58).

5. Implant chirurgical selon l'une quelconque des revendications précédentes, dans lequel ladite surface d`assujettissement (68) comprend une surface incurvée, concave ou multiangulaire afin de correspondre à une anatomie de ladite ou desdites facette externe et/ou surface laminaire (14b) de ladite seconde vertèbre (14).
